## Europäisches Patentamt
## European Patent Office
## Office européen des brevets

(19)

(11) Veröffentlichungsnummer: **0 144 625**
**B1**

(12) # EUROPÄISCHE PATENTSCHRIFT

(45) Veröffentlichungstag der Patentschrift:
**10.08.88**

(51) Int. Cl.⁴: **A 61 M 5/20**

(21) Anmeldenummer: **84112180.9**

(22) Anmeldetag: **11.10.84**

(54) **Injektionsgerät.**

(30) Priorität: **24.11.83 DE 3342407**

(43) Veröffentlichungstag der Anmeldung:
**19.06.85 Patentblatt 85/25**

(45) Bekanntmachung des Hinweises auf die Patenterteilung:
**10.08.88 Patentblatt 88/32**

(84) Benannte Vertragsstaaten:
**AT BE CH DE FR GB IT LI LU NL SE**

(56) Entgegenhaltungen:
**DE-B-1 491 842**
**DE-B-1 929 394**
**DE-B-1 934 117**
**US-A-3 543 603**

(73) Patentinhaber: **Maurer, Erwin, Reichenbucher Strasse 59, D-6950 Mosbach (DE)**

(72) Erfinder: **Maurer, Erwin, Reichenbucher Strasse 59, D-6950 Mosbach (DE)**

(74) Vertreter: **Hach, Hans Karl, Dr., Tarunstrasse 23, D-6950 Mosbach- Waldstadt (DE)**

EP 0 144 625 B1

## Beschreibung

Die Erfindung betrifft ein Injektionsgerät mit einer Injektionsspritze, die koaxial in einem kreiszylindrischen Gehäuse untergebracht ist, welches Gehäuse beidseitig durch Deckel verschlossen ist, von welchen Deckeln der am vorderen Gehäuseende angeordnete einen zentralen Durchbruch für die Injektionskanüle aufweist, der mit einem von der Injektionskanüle durchstoßbaren Pfropfen verschlossen ist,

mit einem Treiber zum Ausfahren der Injektionsnadel durch den Durchbruch und zum Ausstoßen des Spritzeninhaltes durch die ausgefahrene Injektionskanüle, und

mit einer Sperre für den Treiber, die durch eine am hinteren Gehäuseende angeordnete Handhabe lösbar ist,

wobei als Treiber eine Feder vorgesehen ist, die zusammengedrückt vorgespannt einerseits auf der Injektionsspritze und andererseits auf dem Gehäuse abgestützt ist,

wobei für die Sperre ein koaxialer Bolzen vorgesehen ist, der eine Ausnehmung aufweist, wobei in die Ausnehmung mindestens eine Sperrkugel paßt,

wobei die Sperrkugel sich nach vorn auf einer Stütze am Gehäuse und nach außen auf einer Manschette abstützt,

und wobei die Manschette als Gegenlager für die Feder ausgebildet ist und nach hinten auf einem gehäusefesten Anschlag abgestützt ist.

Ein Injektionsgerät dieser Art dient dazu, im Katastrophenfall eine Einmal-Injektion zu verabfolgen (siehe DE-B-1 491 842).

Aufgabe der Erfindung ist es, ein Injektionsgerät der eingangs genannten Art so auszugestalten, daß es in Bereitstellung auch bei rauhem Betrieb nicht versehentlich ausgelöst werden kann und bei Bedarf einfach und sicher ausgelöst werden kann.

Die Erfindung ist dadurch gekennzeichnet, daß der Bolzen am hinteren Ende der spritze befestigt ist,

daß die Manschette nach vorn gegen die Kraftwirkung der Feder verschieblich ist in eine Stellung, in der sie das Austreten der Sperrkugel aus der Ausnehmung nicht behindert, und

daß der hintere Deckel als Handhabe vorgesehen ist und die Manschette mitnehmend nach vorn verschieblich gelagert ist.

Hinter den Sperrkugeln verhakt sich mit der Ringnut die Spritze und wird so gegen die Kraftwirkung der gespannten Feder zurückgehalten. Diese Sperre kann sich auch nicht versehentlich oder durch Erschütterungen selbsttätig lösen, sie kann nur gelöst werden durch Niederdrücken der Handhabe. Das Injektionsgerät zeichnet sich durch einfachen, übersichtlichen Aufbau unter Verwendung weniger einfacher Bauteile aus und gewährleistet betriebssichere Funktion.

Bei einer bevorzugten Ausgestaltung nach Anspruch 3 liefert die Feder für den Injektor auch einen wünschenswerten Gegendruck für die Handhabe.

Die Handhabe wird vor versehentlicher Betätigung zusätzlich gesichert durch eine Ausgestaltung nach Anspruch 4.

Eine Ausgestaltung, die sich dadurch auszeichnet, daß das Gerät einfach montiert, zusammengesetzt und auch wieder zum Füllen auseinandergenommen werden kann, ist insbesondere Gegenstand der Ansprüche 7 und 8.

Eine bevorzugte Ausgestaltung nach Anspruch 9 gestattet es, mit besonders einfachen Mitteln sicherzustellen, daß zuerst die Injektionskanüle und erst dann die Flüssigkeit ausgestoßen wird.

Die Erfindung wird nun anhand der beigefügten Zeichnung näher erläutert.

In der Zeichnung zeigt:

| Figur 1 | ein Injektionsgerät nach der Erfindung mit gefüllter Spritze in Bereitstellung im Längsschnitt, |
|---|---|
| Figur 2 | das gleiche Gerät, nachdem die Sperre gelöst wurde, |
| Figur 3 | den Schnitt III aus Figur 1, |
| Figur 4 | den Schnitt IV aus Figur 1, |
| Figur 5 | ein zweites Ausführungsbeispiel eines Injektionsgerätes mit gefüllter Spritze in Bereitstellung im Längsschnitt, |
| Figur 6 | das Gerät aus Figur 5, nachdem die Sperre gelöst wurde, und |
| Figur 7 | den Schnitt VII aus Figur 5. |

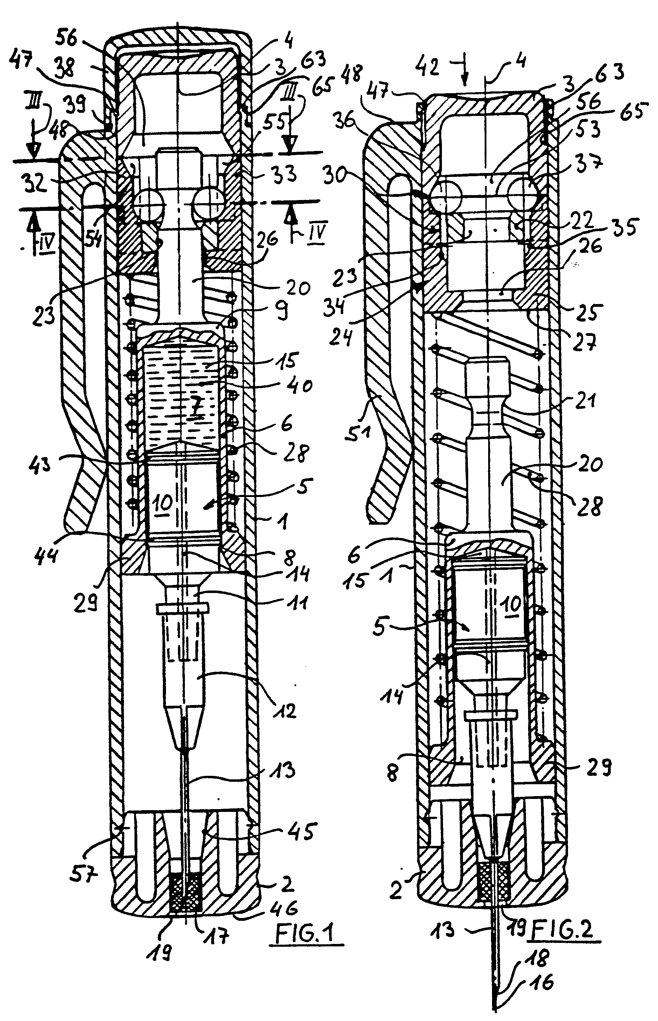

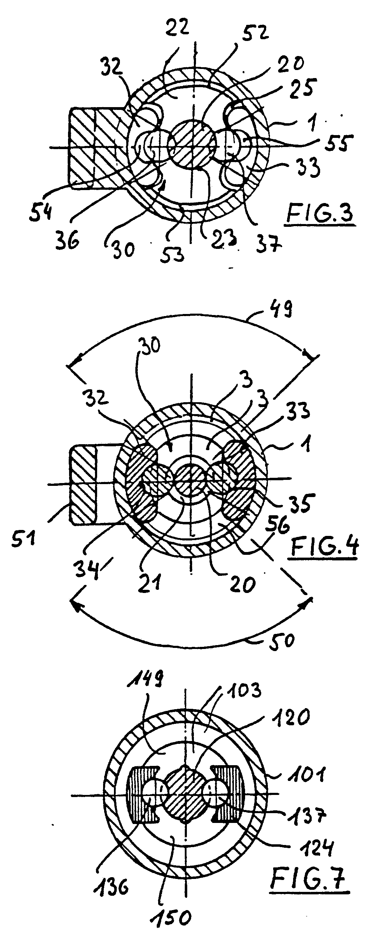

In Figur 1 bis 4 ist mit 1 ein kreiszylinderförmiges Gehäuse bezeichnet, das am vorderen Ende durch einen Deckel 2 und am hinteren Ende durch einen als Handhabe 3 dienenden Deckel verschlossen ist. Beide Deckel sind je durch eine Rast gesichert und können unter Überwindung der Rast abgenommen werden. Die Rast 57 gehört zu dem Deckel 2, ein Wulst 47 am Gehäuse und eine Einschnürung 48 an der Handhabe 3 bilden die Rast für den hinteren Deckel. Koaxial zur Achse 4 ist innerhalb des Gehäuses 1 eine Injektionsspritze 5 angeordnet. Diese Injektionsspritze 5 besteht aus einem Spritzenkörper 6, der einen kreiszylindrischen, koaxialen Flüssigkeitstank 7 aufweist. Der Flüssigkeitstank 7 ist nach vorn, das ist in Figur 1 und 2 unten, offen. Die betreffende Öffnung 8 ist kreisrund. Hinten ist der Flüssigkeitstank 7 durch einen Boden 9 geschlossen. In dem Flüssigkeitstank 7 steckt, passend von vorn eingesteckt, ein kreiszylindrischer Kolben 10, der an seinem vorderen Ende einen Nadelhalter 11 aufweist, auf den eine rückwärtige Aufsetzkappe 12 einer Injektionskanüle 13 aufgesteckt ist. Der Kolben 10 weist einen zur Achse 4 koaxialen Kanal 14 auf, der vom Inneren des Flüssigkeitstanks 7 zur Injektionskanüle führt und mit zwei Abdichtwulsten 43, 44 abgedichtet ist.

Die Injektionskanüle 13 steckt mit ihrer abgeschrägten Spitze 16 in einem Pfropfen 17 aus Dichtmaterial. Der Pfropfen 17 verschließt die vordere Öffnung 18 der Injektionskanüle. Der Pfropfen 17 verschließt außerdem einen zentralen Durchbruch 19, der in dem Deckel 2 vorgesehen ist. Das Material, aus dem der Pfropfen besteht, ist hinreichend abdichtend aber so weich, daß es von der nach vorn ausfahrenden Injektionskanüle durchstoßen werden kann.

Am Boden 9 ist koaxial zur Achse 4 ein Bolzen 20 angeformt, der eine Ringnut 21 aufweist. Dieser Bolzen ist geführt in einem zentralen Durchbruch 23 einer gehäusefest angeordneten Stütze 22.

Mit 24 ist eine Manschette bezeichnet, die eine Bodenplatte 25 aufweist mit einem zentralen Durchbruch 26, durch den der Bolzen 20 gesteckt ist. Auf der Stirnseite 27 der Bodenplatte 25 stützt sich eine in Bereitstellung zusammengedrückte Schraubenfeder 28 ab, die koaxial zur Achse 4 den Spritzen körper 6 umgibt und sich vorn an einem Kragen 29 des Spritzenkörpers 6 abstützt. Der Kragen 29 bildet mit der inneren Wand des Gehäuses 1 eine Führung für die Längsbewegung des Spritzenkörpers 6 in Richtung der Achse 4. Unter der Druckwirkung der Feder 28 stützt sich die Bodenplatte 25 der Manschette 24 an der Stütze 22 ab. Die Manschette 24 weist zwei einander diametral gegenüberstehende Flügel 32, 33 auf und paßt mit ihrer Bodenplatte 25 und diesen Flügeln gleitfähig in Richtung der Achse 4 in das Gehäuse 1. Auf der Innenseite der beiden Flügel 32, 33 ist je ein Paßkanal 34, 35 eingelassen. Die Paßkanäle stehen einander diametral gegenüber und erstrecken sich parallel zur Achse 4.

Mit 36, 37 sind zwei gleichgroße Sperrkugeln bezeichnet, die in die Ringnut 21 ragen mit etwa 1/4 ihres Durchmessers.

Nach unten stützen sich die Sperrkugeln auf der Stütze 22 ab und quer dazu nach außen auf den Flügeln 32, 33. Zwischen den Flügeln 32, 33 erstrecken sich zwei Schlitze 49, 50 der Manschette 24, die bis an das rückwärtige Ende der Manschette reichen und dort offen sind. Durch diese Schlitze ragt die Stütze 22 wie aus Figur 2 ersichtlich bis an das Gehäuse 1 und ist dort verankert.

Die Sperrkugeln ragen in die Paßkanäle 34, 35, die sich vom oberen Ende der Manschette 24 bis auf die Höhe der in Sperrstellung gemäß Figur 1 befindlichen Sperrkugeln 36, 37 erstrecken. Die Sperrkugeln hindern, solange sie in die Ringnut 21 ragen, den Bolzen 20 der Federkraft der gespannten Feder 28 folgend nach vorn auszuweichen. Sie halten die Spritze 5 vielmehr sicher in der in Figur 1 gezeichneten Sperrstellung und bilden mit der Manschette 24 und dem Bolzen 20 eine allgemein mit 30 bezeichnete Sperre.

Die als Deckel ausgebildete Handhabe 3 ragt oben aus dem Gehäuse 1 heraus und steckt in Achsrichtung 4 verschieblich im Gehäuse 1. Die Handhabe 3 ist durch nach innen vorspringende Anschläge 47, 63 des Gehäuses und entsprechende Vorsprünge 48, 65 an der Handhabe daran gehindert, nach hinten auszuweichen. Sie sitzt in der in Figur 1 gezeichneten Sperrstellung mit ihrem vorderen Rand auf dem rückwärtigen Rand der Flügel 32, 33 der Manschette 24.

Die rückwärtigen Enden der Paßkanäle 34, 35 sind Erweiterungen 54, 55. Am gegenüberliegenden vorwärtigen Ende der Handhabe 3 ist eine entsprechende Erweiterung 56 vorgesehen. Die Erweiterungen 54, 55 bieten bei nach vorn geschobener Handhabe 3 Platz für die Sperrkugeln, so daß diese aus der Ringnut 21 austreten können. Über die Handhabe 3 ist eine Schutzkappe 38 gestülpt, die mit einer Rast 39 gesichert auf das hintere Ende des Gehäuses gesteckt ist.

Mit 51 ist ein Halteklipp bezeichnet, der es gestattet, das in Bereitstellung befindliche Injektionsgerät, das etwa die Größe eines groben Füllfederhalters hat, wie einen Füllfederhalter eingesteckt in einer Jackentasche vor dem Verlieren zu sichern.

In Bereitstellung ist der Flüssigkeitstank 7 und der Kanal 14 sowie das Innere der Injektionskanüle 13 mit Injektionsflüssigkeit 40 gefüllt. Wenn eine Injektion verabfolgt werden soll, wird die Schutzkappe 38 abgezogen und das Gehäuse 1 mit der vorderen Stirnfläche 46 des vorderen Deckels 2 auf die Haut aufgesetzt - im Notfall unter Zwischenlage der Garderobeund dann wird die Handhabe 3 in Pfeilrichtung 42 nach vorn gedrückt. Dadurch wird die Manschette 24 gegen die Kraftwirkung der Feder 28 ein Stück nach unten geschoben in die in Figur 2 gezeichnete Stellung, in der die Sperrkugeln 36, 37 nach außen aus der Ringnut 21 ausweichen können in die ringförmigen Erweiterungen 54, 55. Sobald die Sperrkugeln die Ringnut 21 verlassen haben, wird die Spritze 5 nicht mehr gehalten und durch die Feder 28 nach vorn getrieben. Dabei durchstößt zunächst die Injektionskanüle 13 den Pfropfen 17 und tritt an dem Durchbruch 19 aus und dringt durch die Haut in das Muskelfleisch ein, bis die Aufsetzkappe 12 in einer Konusöffnung 45 des Deckels 2 am Deckel 2 anschlägt. Der Kolben 10 kann nun nicht mehr weiter nach vorn ausweichen. Da aber der Spritzenkörper 6 noch weiter nach vorn getrieben wird, wird nun die Flüssigkeit 40 durch die Injektionskanüle 13 unter der Kraftwirkung der Feder 28 ausgetrieben und in das Muskelfleisch gespritzt, bis der Spritzenkörper am Kolben 10 anschlägt in seiner in Figure 2 gezeichneten Endstellung.

Wenn das Injektionsgerät nach durchgeführter Injektion erneut verwendet werden soll, wird zum Verankern des Bolzens 20 in der Sperre 30 die Spritze 5 unter gleichzeitigem Spannen der Feder 28 nach hinten gedrückt, und zwar über die Bereitstellung hinaus. In dieser Stellung können die Sperrkugeln 36, 37 von oben in ihre Sperrstellung einlaufen, während das Injektionsgerät mit dem hinteren Ende nach oben gehalten wird.

Wenn man nun die Spritze 5 losläßt, weicht sie unter der Kraftwirkung der Feder 28 in die in Figur 1 gezeichnete Sperrstellung. In entsprechender Weise wird das Gerät auch bei der Erstmontage zusammengesetzt. Gegebenenfalls kann dabei die Handhabe 3 abgenommen werden, um die Sperrkugeln sicher in die Paßkanäle 34, 35 einzuführen. Man kann nun die geleerte und gesäuberte Injektionsspritze 5 bei herausgezogenem Kolben 10 desinfizieren. Dann wird das Innere 15 des Flüssigkeitstanks mit Injektionsflüssigkeit 40 gefüllt und der Kolben 10 eingesteckt, wobei gleichzeitig das Gerät mit dem vorderen Ende nach oben gehalten wird, so daß eine luftblasenfreie Füllung einschließlich der Injektionskanüle gesichert ist. Dann wird der Deckel 2 aufgesetzt und der Pfropfen 17 eingespritzt. Das Injektionsgerät ist nun wieder in Bereitstellung.

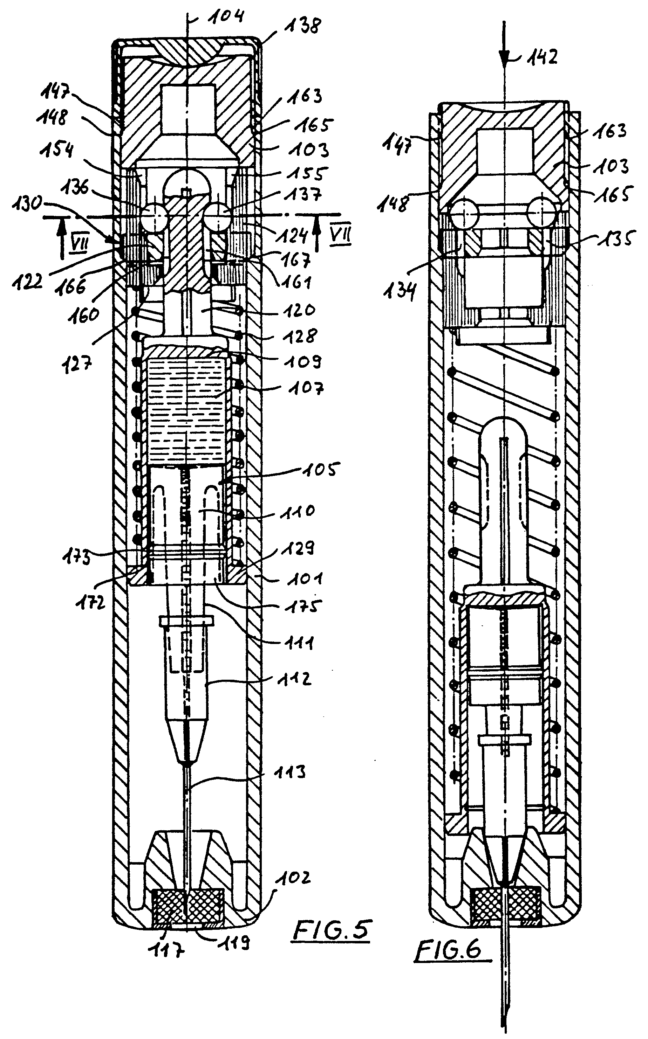

Das Ausführungsbeispiel nach Figur 5 bis 7 ist dem Ausführungsbeispiel nach Figur 1 bis 4 sehr ähnlich. Es werden nachfolgend vorwiegend nur die Unterschiede beschrieben und soweit nicht beschrieben ist das Ausführungsbeispiel nach Figur 5 bis 7 genauso ausgebildet wie das Ausführungsbeispiel nach Figur 1 bis 4 und wird auch genauso betrieben.

Nach Figur 5 bis 7 ist mit 101 das rohrförmige Gehäuse bezeichnet, das vorn durch einen angeformten Deckel 102 verschlossen ist und hinten durch einen als Handhabe 103 dienenden Deckel verschlossen werden kann. Mit 105 ist eine Injektionsspritze bezeichnet, deren Spritzenkörper mit 106, deren Flüssigkeitstank mit 107, deren Kolben mit 110, deren Nadelhalter mit 111, deren Aufsetzkappe mit 112 und deren Injektionsnadel mit 113 bezeichnet ist. Der Kolben 110 greift zur Abdichtung mit einem Wulst 172 in eine Ringnut 173 des Spritzenkörpers 106. Der Kolben weist vorn eine Ausnehmung 175 auf, die den Nadelhalter 111 umgibt, um den Nadelhalter zu verfedern und den Kolben vom Gewicht zu entlasten.

Mit 129 ist ein Kragen am vorderen Ende des Spritzenkörpers 106 bezeichnet, auf dem sich eine gemäß Figur 5 zusammengedrückt gespannte Schraubenfeder 128 abstützt. Am rückwärtigen Ende des Spritzenkörpers 106 ist koaxial zur Achse 104 des Gehäuses ein Bolzen 120 befestigt, der diametral einander gegenüberliegend zwei langgestreckte Ausnehmungen 160, 161 aufweist, die sich parallel zur Achse 104 erstrecken und in die zwei Sperrkugeln 136, 137 passen. Mit 124 ist eine Manschette bezeichnet, auf deren Stirnseite 127 sich nach Figur 5 die Schraubenfeder 128 abstützt. Die Manschette 124 ihrerseits stützt sich nach hinten auf der Handhabe 103 ab, die sich ihrerseits mit zwei einander diametral gegenüberliegenden Vorsprüngen 148, 165 auf zwei diametral gegenüberliegenden, gehäusefesten Anschlägen 147, 163 nach hinten abstützt. Die Handhabe 103 kann nach Art eines Bajonett-Verschlusses gelöst werden, indem sie um 90 Grad, bezogen auf die Achse 104, im Gehäuse gedreht wird. Dann sind die Vorsprünge 148, 165 frei von den Anschlägen 147, 163 und finden Platz in Kanälen des Gehäuses, die in der Zeichnung nicht sichtbar sind, so daß in der gegenüber der Zeichnung um 90 Grad verdrehten Stellung der Handhabe diese nach oben aus dem Gehäuse herausgezogen werden kann.

Die Manschette 124 weist diametral einander gegenüberliegend zwei Paßkanäle 134, 135 auf, in die die Sperrkugeln 136, 137 gemäß Figur 5 passen. Die Sperrkugeln stützen sich nach vorn gemäß Figur 5 auf einer Stütze 122 ab, die den Bolzen 120 umgibt und die Manschette 124 durchsetzt. Diese Stütze 122 ist ein loses Teil, das nach vorn beidseitig an einem gehäusefesten Anschlag 166, 167 abgestützt ist. Die Injektionsspritze 105 stützt sich gemäß Figur 5 gegen die Kraftwirkung der Schraubenfeder 128 über die Sperrkugeln 136, 137 und über die Stütze 122 an den Anschlägen 166, 167 ab. Die Sperrkugeln 136, 137 können nicht ausweichen, da sie nach Figur 5 formschlüssig in die Ausnehmungen 160, 161 und die Paßkanäle 134, 135 passen. Die so gebildete Sperre ist allgemein mit 130 bezeichnet. Die Stütze 122 durchsetzt die Manschette in zwei Schlitzen 149, 150, die bis an das rückwärtige Ende der Manschette reichen.

Mit 138 ist eine lose aufgesetzte, abziehbare Schutzkappe bezeichnet, die sich aufgesetzt auf dem Gehäuse 101 abstützt und stabil genug ist, um als Sicherung gegen unbeabsichtigtes Niederdrücken der Handhabe 103 zu dienen. Mit 117 ist ein von der Injektionsnadel 113 durchstoßbarer Pfropfen bezeichnet, der einen Durchbruch 119 im Deckel 102 gemäß Figur 5 verschließt. Mit 154, 155 sind zwei Erweiterungen am rückwärtigen Ende der Paßkanäle 134, 135 bezeichnet, in denen die Sperrkugeln 136, 137 genügend Platz finden, um die Injektionsspritze 105 freizugeben.

Zur Auslösung des Spritzvorganges wird die Schutzkappe 138 entfernt und die Handhabe 103 in Pfeilrichtung 142 gegen die Kraftwirkung der gespannten Schraubenfeder 128 nach vorn gedrückt. Dadurch verschiebt sich die Manschette 124 nach vorn. Die Sperre 130 bleibt stehen und findet Platz in den Schlitzen 149, 150 der Manschette 124. Diese Schlitze erstrecken sich parallel zur Achse 104. Beim Vorschub der Manschette 124 geraten die Sperrkugeln schließlich in den Bereich der Erweiterungen 154, 155 und können nach außen ausweichen und geben dadurch den Bolzen 120 frei. Die Injektionsspritze 105 wird nun durch die Feder 128 nach vorn getrieben bis die Aufsetzkappe 112 auf dem Deckel 102 anschlägt. Die Injektionsnadel 113 hat bis dahin den Pfropfen 117 durchsetzt, wie aus Figur 6 ersichtlich. Die Schraubenfeder 128 treibt nun den Spritzenkörper 106 weiter nach vorn, nachdem sie die Rastwirkung des Wulstes 172 in der Ringnut 173 überwunden hat, bis der Kolben 110 auf dem Boden 109 anschlägt, wobei die Injektionsflüssigkeit aus dem Flüssigkeitstank 107 ausgetrieben wird.

Zum Wiederbefüllen wird die Handhabe 103 durch Verdrehen um 90 Grad gelöst und nach hinten abgezogen. Ist das geschehen, dann können sämtliche Teile, die sich innerhalb des Gehäuses 101 befinden, nach hinten herausgezogen werden. Die Injektionsspritze 105 wird dann gefüllt, es wird ein neuer Pfropfen 117 eingesetzt und die Teile werden von hinten wieder eingeführt, und zwar zunächst die gefüllte Injektionsspritze 105 mit der Manschette 124, der in die Manschette eingesteckten Sperre 130 mit in Sperrstellung befindlichen Sperrkugeln 136, 137 und der dazwischen vorgespannten Schraubenfeder 128. Dann wird die Handhabe 103 aufgesetzt und dabei die Schraubenfeder 128 etwas nachgespannt und dann wird die Handhabe 103 durch Verdrehen um 90 Grad an den Anschlägen 147, 163 gesichert und die Schutzkappe 138 wird aufgesetzt. In entsprechender Weise erfolgt auch die Montage.

**Patentansprüche**

1. Injektionsgerät mit einer Injektionsspritze, die koaxial in einem kreiszylindrischen Gehäuse untergebracht ist, welches Gehäuse beidseitig durch Deckel verschlossen ist, von welchen Deckeln der am vorderen Gehäuseende angeordnete einen zentralen Durchbruch für die Injektionskanüle aufweist, der mit einem von der Injektionskanüle durchstoßbaren Pfropfen verschlossen ist,

mit einem Treiber zum Ausfahren der Injektionsnadel durch den Durchbruch und zum Ausstoßen des Spritzeninhaltes durch die ausgefahrene Injektionskanüle, und

mit einer Sperre für den Treiber, die durch eine am hinteren Gehäuseende angeordnete Handhabe lösbar ist,

wobei als Treiber eine Feder (28, 128) vorgesehen ist, die zusammengedrückt vorgespannt einerseits auf der Injektionsspritze (5, 105) und andererseits auf dem Gehäuse (1, 101) abgestützt ist,

wobei für die Sperre (30, 130) ein koaxialer Bolzen (20, 120) vorgesehen ist, der eine Ausnehmung (21, 160, 161) aufweist,

wobei in die Ausnehmung mindestens eine Sperrkugel (36, 37, 136, 137) paßt,

wobei die Sperrkugel sich nach vorn auf einer Stütze (22, 122) am Gehäuse und nach außen auf einer Manschette (24, 124) abstützt,

und wobei die Manschette als Gegenlager für die Feder ausgebildet ist und nach hinten auf einem gehäusefesten Anschlag (22, 147) abgestützt ist,

dadurch gekennzeichnet,

daß der Bolzen (20, 120) am hinteren Ende der Spritze (5, 105) befestigt ist,

daß die Manschette nach vorn gegen die Kraftwirkung der Feder verschieblich ist in eine Stellung, in der sie das Austreten der Sperrkugel aus der Ausnehmung nicht behindert, und

daß der hintere Deckel (3, 103) als Handhabe vorgesehen ist und die Manschette mitnehmend nach vorn verschieblich gelagert ist.

2. Gerät nach Anspruch 1, dadurch gekennzeichnet,

daß die Feder (28, 128) eine Schraubenfeder ist, die koaxial die Spritze (5, 105) umgebend angeordnet ist und sich einerseits am vorderen Ende der Spritze und andererseits an der vorderen Stirnseite (27, 127) der Manschette (24, 124) abstützt.

3. Gerät nach Anspruch 1 oder 2, dadurch gekennzeichnet, daß die Manschette (24, 124) nach hinten auf der Handhabe (3, 103) abgestützt ist, und

daß die Handhabe (3, 103) nach hinten auf dem gehäusefesten Anschlag (47, 147) abgestützt ist und nach vorn gegen die Kraftwirkung der Feder (28, 128) verschieblich ist.

4. Gerät nach einem oder mehreren der vorhergehenden Ansprüche, dadurch gekennzeichnet,

daß die Handhabe (3, 103) durch eine auf das hintere Gehäuseende aufsetzbare Schutzkappe (38, 138) abdeckbar ist.

5. Gerät nach einem oder mehreren der vorhergehenden Ansprüche, dadurch gekennzeichnet,

daß an dem Bolzen (20, 120) ein oder mehrere Ausnehmungen (21, 160, 161) für zwei einander diametral gegenübergelegene Sperrkugeln (36, 37, 136, 137) vorgesehen sind.

6. Gerät nach Anspruch 5, dadurch gekennzeichnet,

daß die Manschette (24, 124) zwei einander diametral gegenüberliegende Paßkanäle (34, 35, 134, 135) für je eine Sperrkugel (36, 37, 136, 137) aufweist,

daß diese Paßkanäle sich achsparallel erstrecken vom hinteren Ende der Manschette (24, 124) bis zu den in Sperrstellung befindlichen Sperrkugeln, und

daß die Manschette zwischen den Paßkanälen Schlitze (49, 50, 149, 150) aufweist, durch die die Stütze (22, 122) ragt.

7. Gerät nach einem oder mehreren der vorhergehenden Ansprüche, dadurch gekennzeichnet,

daß die Stütze (122) ein loses Teil ist, das nach vorn auf einem gehäusefesten Anschlag (166, 167) abgestützt ist.

8. Gerät nach einem oder mehreren der vorhergehenden Ansprüche, dadurch gekennzeichnet,

daß die Handhabe (103) durch Verdrehen von ihrem rückwärtigen Anschlag (147, 163) abgesetzt und verdreht nach rückwärts aus dem Gehäuse (101) herausziehbar ist, und

daß die Injektionsspritze (105), die Feder (128), die Stütze (122) und die Sperrkugeln (136, 137) bei entfernter Handhabe (103) nach hinten aus dem Gehäuse herausziehbar sind.

9. Gerät nach einem oder mehreren der vorhergehenden Ansprüche, dadurch gekennzeichnet,

daß die Spritze (5, 105) aus einem Spritzenkörper (6, 106) besteht, der einen kreiszylindrischen, koaxialen Flüssigkeitstank (7, 107) aufweist, der nach vorn offen und nach hinten geschlossen ist, und

daß in den Flüssigkeitstank von vorn ein Kolben (10, 110) eingesteckt ist, der an seinem vorderen Ende die Injektionsnadel (13, 113) trägt und einen zur Injektionsnadel führenden, koaxialen Kanal (14, 114) aufweist.

10. Gerät nach einem oder mehreren der vorhergehenden Ansprüche, dadurch gekennzeichnet,

daß der Kolben (110) der Injektionsspritze (105) einen ringförmigen Abdichtwulst (172) aufweist, der bei gefüllter Injektionsspritze in eine Ringnut (173) innen am Spritzenkörper (106) einrastet und eine Abdichtung für die aufgezogene Injektionsflüssigkeit bildet.


## Claims

1. Injection apparatus with an injection syringe, which is coaxially housed in a circular cylindrical casing, said casing being closed on both sides by covers, the cover located on the front casing end having a central passage for the injection cannula, which is closed by a stopper penetratable by the injection cannula, with a driver for extending the injection needle through the opening and for ejecting the syringe content through the extended injection cannula and with a locking device for the driver, which can be released by a handle arranged on the rear casing end, in which the driver is constituted by a spring (28, 128), which compressed is supported in pretensioned manner on the one hand on the injection syringe (5, 105) and on the other on casing (1, 101), in which for the locking device (30, 130) is provided a coaxial bolt (20, 120) having a recess (21, 160, 161), in which at least one locking ball (36, 37, 136, 137) fits into the recess, in which the locking ball is forwardly supported on a support (22, 122) on the casing and outwardly supported on a sleeve (24, 124) and in which the sleeve is constructed as an abutment for the spring and is rearwardly supported on a casing-fixed stop (22, 147), characterized in that the bolt (20, 120) is fixed to the rear end of the syringe (5, 105), that the sleeve is forwardly displaceable counter to the tension action of the spring into a position in which it does not impede the escape of the locking ball from the recess and that the rear cover (3, 103) is provided as a handle and is forwardly displaceably mounted carrying the sleeve with it.

2. Apparatus according to claim 1, characterized in that the spring (28, 128) is a helical spring, which coaxially surrounds the syringe (5, 105) and is supported on the one hand on the front end of the syringe and on the other on the front end face (27, 127) of sleeve (24, 124).

3. Apparatus according to claims 1 or 2, characterized in that the sleeve (24, 124) is rearwardly supported on handle (3, 103) and that the handle (3, 103) is rearwardly supported on the casing-fixed stop (47, 147) and is forwardly displaceable against the tension action of spring (28, 128).

4. Apparatus according to one or more of the preceding claims, characterized in that the handle (3, 103) can be covered by a protective cap (38, 138) which can be placed on the rear casing end.

5. Apparatus according to one or more of the preceding claims, characterized in that on bolt (20, 120) are provided one or more recesses (21, 160, 161) for two diametrically facing locking balls (36, 37, 136, 137).

6. Apparatus according to claim 5, characterized in that the sleeve (24, 124) has two diametrically facing fitting ducts (34, 35, 134, 135) for in each case one locking ball (36, 37, 136, 137), that these fitting ducts extend in axially parallel manner from the rear end of sleeve (24, 124) to the locking balls located in the locking position and that the sleeve is provided between the fitting ducts with slots (49, 50, 149, 150) through which projects support (22, 122).

7. Apparatus according to one or more of the preceding claims, characterized in that the support (122) is a loose part, which is forwardly supported on a casing-fixed stop (166, 167).

8. Apparatus according to one or more of the preceding claims, characterized in that the handle (103) is removed from its rear stop (147, 163) by rotation and rotated rearwards can be drawn out of casing (101) and that the injection syringe (105), spring (128), support (122) and locking balls (136, 137) can be rearwardly drawn out of the casing with handle (103) removed.

9. Apparatus according to one or more of the preceding claims, characterized in that the syringe (5, 105) comprises a syringe body (6, 106) having a circular cylindrical, coaxial liquid tank (7, 107), which is forwardly open and rearwardly closed and that from the front a plunger (10, 110) is inserted in the liquid tank, said plunger carrying at its front end the injection needle (13, 113) and has a coaxial duct (14, 114) leading to the injection needle.

10. Apparatus according to one or more of the preceding claims, characterized in that the plunger (110) of injection syringe (105) has an annular sealing bead (172) which, when the injection syringe is filled, engages in an annular groove (173) on the inside of the syringe body (106) and forms a seal for the drawn on injection fluid.


## Revendications

Dispositif à injection, muni d'une seringue d'injection logée coaxialement dans un boîtier de forme cylindrique droite, ce boîtier étant obturé de part et d'autre par des couvercles, couvercles parmi lesquels celui situé à l'extrémité antérieure du boîtier est percé d'un évidement central pour la canule d'injection, qui est obturé par un bouchon perforable par la canule d'injection; d'un élément d'entraînement pour sortir l'aiguille d'injection à travers l'évidement, et pour expulser le contenu de la seringue à travers la canule d'injection sortie; et d'un moyen de verrouillage pour l'élément d'entraînement, qui peut être déclenché par l'intermédiaire d'une partie de préhension disposée à l'extrémité postérieure du boîtier,

l'élément d'entraînement étant matérialisé par un ressort (28, 128) qui, comprimé avec précharge, prend appui d'une part sur la seringue d'injection (5, 105) et d'autre part sur le boîtier (1, 101),

une cheville coaxiale (20, 120), prévue pour le moyen de verrouillage (30, 130), étant pourvue d'une dépouille (21, 160, 161),

au moins une bille de blocage (36, 37, 136, 137) s'ajustant dans la dépouille,

la bille de blocage prenant appui, vers l'avant, sur une pièce de soutien (22, 122) installée sur le boîtier et, vers l'extérieur, sur une douille (24, 124),

et la douille étant réalisée en tant que contrebutée pour le ressort et étant en appui, vers l'arrière, sur une butée (22, 147) assujettie au boîtier,

caractérisé par le fait que la cheville (20, 120) est fixée à l'extrémité postérieure de la seringue (5, 105),

que la douille peut coulisser vers l'avant, en s'opposant à l'action de la force du ressort, jusqu'à une position dans laquelle elle ne gène pas la sortie de la bille de blocage hors de la dépouille, et

que le couvercle postérieur (3, 103) est prévu en tant que partie de préhension et est monté avec faculté de coulissement vers l'avant, en entraînant la douille.

2. Dispositif selon la revendication 1, caractérisé par le fait

que le ressort (28, 128) est un ressort hélicoïdal qui est implanté de manière à ceinturer coaxialement la seringue (5, 105) et qui prend appui, d'une part, contre l'extrémité antérieure de cette seringue et, d'autre part, contre la face extrême antérieure (27, 127) de la douille (24, 124).

3. Dispositif selon la revendication 1 ou 2, caractérisé par le fait

que la douille (24, 124) prend appui vers l'arrière sur la partie de préhension (3, 103), et que la partie de préhension (3, 103) prend appui vers l'arrière sur la butée (47, 147) assujettie au boîtier, et peut coulisser vers l'avant en s'opposant à l'action de la force du ressort (28, 128).

4. Dispositif selon l'une ou plusieurs des revendications précédentes, caractérisé par le fait

que la partie de préhension (3, 103) peut être coiffée par un capuchon protecteur (38, 138), qui peut être enfilé sur l'extrémité postérieure du boîtier.

5. Dispositif selon l'une ou plusieurs des revendications précédentes, caractérisé par le fait

que, sur la cheville (20, 120), une ou plusieurs dépouilles (21, 160, 161) sont prévues pour deux billes de blocage (36, 37, 136, 137) diamétralement opposées l'une à l'autre.

6. Dispositif selon la revendication 5, caractérisé par le fait

que la douille (24, 124) présente deux logements d'ajustement (34, 35, 134, 135) diamétralement opposés l'un à l'autre, destinés chacun à une bille respective de blocage (36, 37, 136, 137),

que ces logements d'ajustement s'étendent parallèlement à l'axe, de l'extrémité postérieure de la douille (24, 124) jusqu'aux billes de blocage occupant la position de blocage, et

que la douille possède, entre les logements d'ajustement, des fentes (49, 50, 149, 150) à travers lesquelles la pièce de soutien (22, 122) dépasse.

7. Dispositif selon l'une ou plusieurs des revendications précédentes, caractérisé par le fait

que la pièce de soutien (122) est une pièce mobile avec jeu, qui prend appui vers l'avant sur une butée (166, 167) assujettie au boîtier.

8. Dispositif selon l'une ou plusieurs des revendications précédentes, caractérisé par le fait

que la partie de préhension (103) peut être extraite du boîtier (101) avec décalage et rotation vers l'arrière, par rotation de sa butée postérieure (147, 163), et

que la seringue d'injection (105), le ressort (128), la pièce de soutien (122) et les billes de blocage (136, 137) peuvent être extraits du boîtier, vers l'arrière, lorsque la partie de préhension (103) est enlevée.

9. Dispositif selon l'une ou plusieurs des revendications précédentes, caractérisé par le fait

que la seringue (5, 105) consiste en un corps de seringue (6, 106) muni d'un réservoir coaxial (7, 107) à fluide, de forme cylindrique droite, qui est ouvert vers l'avant et fermé vers l'arrière, et qu'un piston (10, 110), emboîté par l'avant dans le réservoir à fluide, porte à son extrémité antérieure l'aiguille d'injection (13, 113) et présente un canal coaxial (14, 114) qui gagne cette aiguille d'injection.

10. Dispositif selon l'une ou plusieurs des revendications précédentes, caractérisé par le fait

que le piston (110) de la seringue d'injection (105) comporte un bourrelet annulaire d'étanchement (172) qui, lorsque la seringue d'injection est emplie, pénètre intérieurement par déclic dans une gorge annulaire (173) ménagée sur le corps de seringue (106), et assure une étanchéité pour le fluide d'injection appelé.

FIG.1

FIG.2

FIG.3

FIG.4

FIG.7

0 144 625

FIG.5

FIG.6

5